# EUROPEAN PATENT APPLICATION

(11) **EP 3 225 173 A1**
(43) Date of publication of application: **04.10.2017**
(21) Application number: 17161733.5
(22) Date of filing: 20.03.2017
(51) Int. Cl.: A61B 17/00, A61M 5/19, A61M 5/315, B05B 7/04, B01F 13/00, A61M 5/31

(54) **APPLICATOR**

(30) Priority: 31.03.2016 JP 2016072507
(71) Applicant: Terumo Kabushiki Kaisha, Shibuya-ku Tokyo (JP)
(72) Inventor: Kai, Miho, Kanagawa, Kanagawa 259-0151 (JP); Fukui, Kunio, Kanagawa, Kanagawa 259-0151 (JP); Isihara, Hiroyuki, Kanagawa, Kanagawa 259-0151 (JP); Souma, Yuuki, Kanagawa, Kanagawa 259-0151 (JP); Chino, Naotaka, Kanagawa, Kanagawa 259-0151 (JP)
(74) Representative: KIPA AB

(57) **Abstract**

There is provided an applicator for ejecting together with a gas a mixed liquid obtained by mixing a first liquid and a second liquid which are different from each other in composition. The applicator includes a first liquid flow path through which the first liquid flows; a second liquid flow path through which the second liquid flows; a gas flow path through which the gas flows; a confluence section in which the first liquid and the second liquid merge with each other to form the mixed liquid, at least part of a wall portion defining the confluence section being composed of a gas-permeable membrane which is impermeable to the mixed liquid and permeable to the gas; and a backflow preventing section that prevents backflow of the first liquid and prevents backflow of the second liquid.

## Description

### BACKGROUND

The present disclosure relates to an applicator.

Heretofore, there has been known a method of mixing two or more liquids and jetting the mixed liquid to an affected area or the like, to form an anti-adhesion material or a living body tissue adhesive. In addition, applicators for use in such a method have been developed.

Such an applicator is configured in such a manner that solutions each containing components capable of coagulating when mixed together, for example, a solution containing thrombin and a solution containing fibrinogen are sent to the vicinity of an affected area in the state of being separate from each other, and the solutions are applied to the affected area while being mixed together.

Conventional applicators include one that has two syringes each containing different liquids, and a nozzle for mixing the liquids from the syringes and ejecting the mixed liquid (refer to, for example, Japanese Patent Laid-open No. 2002-282368).

In addition, the applicator disclosed in Japanese Patent Laid-open No. 2002-282368 has a configuration wherein the nozzle is connected to a gas source for supplying an aseptic gas, and the liquid is ejected together with the aseptic gas. The nozzle specifically has a double tube structure composed of two inner tubes through which the liquids from the syringes each pass, and an outer tube in which the two inner tubes are inserted such that the gas passes through a space between the outer tube and the inner tubes. Each of the inner tubes has its distal opening functioning as a liquid ejection port for ejecting the liquid. A distal opening of the outer tube functions as a gas ejection port inside of which the liquid ejection ports are disposed and through which the gas is ejected.

In the case of the nozzle configured in this way, the liquids ejected outward through the liquid ejection ports of the inner tubes are distributed also to the gas ejection port. Therefore, the liquids may mix with each other and thereby coagulate also at the gas ejection port, leading to clogging of the gas ejection port. If it is attempted to again perform an applying operation by use of the applicator wherein the clogging has thus occurred, the coagulated liquid obstructs the ejection of the liquids from the liquid ejection ports and the ejection of the gas from the gas ejection port. In such a situation, the applying operation cannot be performed again.

### SUMMARY

There is a need for an applicator with which it is possible to prevent a first liquid flow path and a second liquid flow path from being clogged.

In one mode of the present disclosure, there is provided an applicator for ejecting together with a gas a mixed liquid obtained by mixing a first liquid and a second liquid which are different from each other in composition, the applicator including:
a first liquid flow path through which the first liquid flows;
a second liquid flow path through which the second liquid flows;
a gas flow path through which the gas flows;
a confluence section in which the first liquid and the second liquid merge with each other to form the mixed liquid, at least part of a wall portion defining the confluence section being composed of a gas-permeable membrane which is impermeable to the mixed liquid and permeable to the gas; and
a backflow preventing section that prevents backflow of the first liquid and prevents backflow of the second liquid.

In the applicator as above, preferably, the backflow preventing section includes check valves which are each provided in the first liquid flow path and the second liquid flow path.

Preferably, the applicator as above further includes:
a first liquid supply section that communicates with the first liquid flow path and supplies the first liquid into the first liquid flow path; and
a second liquid supply section that communicates with the second liquid flow path and supplies the second liquid into the second liquid flow path,
wherein the check valves are each provided in a boundary section between the first liquid flow path and the first liquid supply section and in a boundary section between the second liquid flow path and the second liquid supply section.

In the applicator as above, preferably, the check valve has a diaphragm that is openable and closable.

In the applicator as above, preferably, the check valve has a biasing section that biases the diaphragm.

In the applicator as above, preferably,
the length of the gas-permeable membrane in a direction in which the mixed liquid flows through the confluence section is 5.0 to 39.0 mm, and
the flow rate of the gas permeating through the gas-permeable membrane is 0.3 to 2.4 L/minute.
In the applicator as above, preferably, the thickness of the gas-permeable membrane is 0.2 to 0.8 mm.

In the applicator as above, preferably, the gas-permeable membrane is tubular in shape with an inside diameter of 0.7 to 1.5 mm.

In the applicator as above, preferably, an outer surface of the gas-permeable membrane has a surface area of 10 to 250 mm².

In the applicator as above, when an applying operation is conducted or when the applying operation is stopped, the gas flows into the confluence section and, due to the gas pressure, the mixed liquid would tend to flow back. In the applicator disclosed herein, however, the backflow preventing section for preventing backflow of the first liquid and the second liquid is provided, and, therefore, the mixed liquid is prevented from flowing back into the first liquid flow path and the second liquid flow path. Accordingly, the first liquid flow path and the second liquid flow path can be prevented from being clogged. As a result, after an applying operation is stopped, the next applying operation can be performed smoothly and reliably.

### BRIEF DESCRIPTION OF THE DRAWINGS

FIG. 1 is a perspective view showing an embodiment of an applicator disclosed herein;
FIG. 2 is a longitudinal sectional view of a nozzle possessed by the applicator shown in FIG. 1;
FIGS. 3A and 3B are longitudinal sectional views of a check valve possessed by the applicator shown in FIG. 1, wherein FIG. 3A shows a closed state and FIG. 3B shows an open state;
FIG. 4 is an enlarged detailed view of a distal portion of the nozzle depicted in FIG. 2, showing variations in an application state with the lapse of time;
FIG. 5 is an enlarged detailed view of the distal portion of the nozzle depicted in FIG. 2, showing variations in the application state with the lapse of time;
FIG. 6 is an enlarged detailed view of the distal portion of the nozzle depicted in FIG. 2, showing variations in the application state with the lapse of time; and
FIG. 7 is an enlarged detailed view of the distal portion of the nozzle depicted in FIG. 2, showing variations in the application state with the lapse of time.

### DETAILED DESCRIPTION OF THE PREFERRED EMBODIMENT

An applicator according to the present disclosure will be described in detail below, based on a preferred embodiment thereof illustrated in the accompanying drawings.

### <Embodiment>

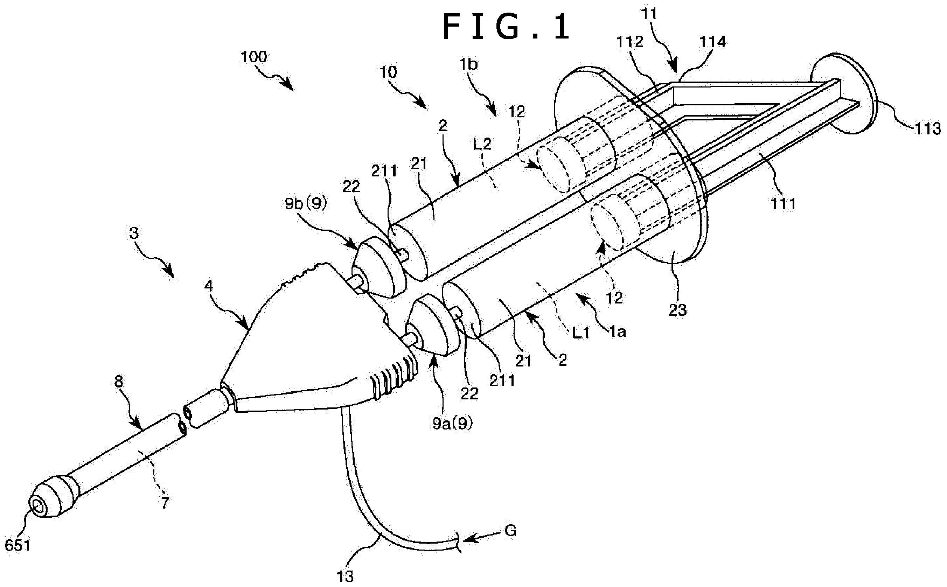

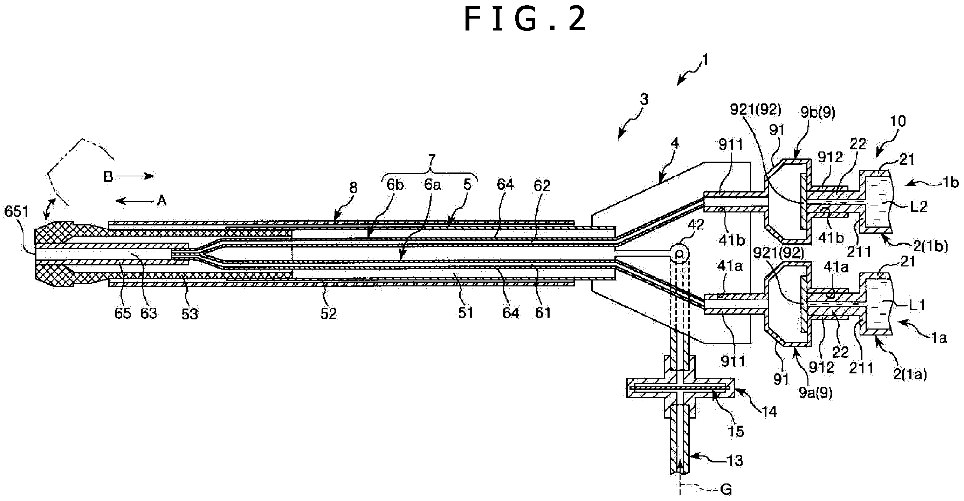

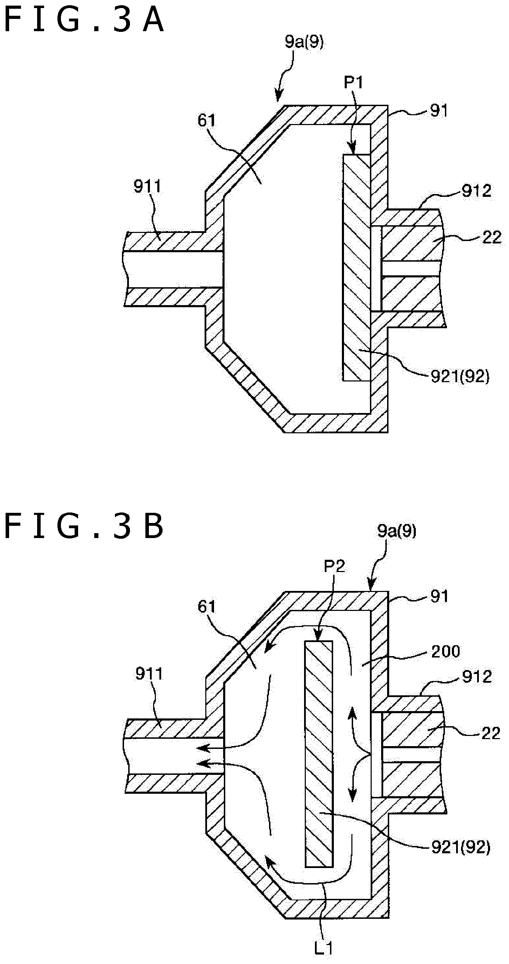

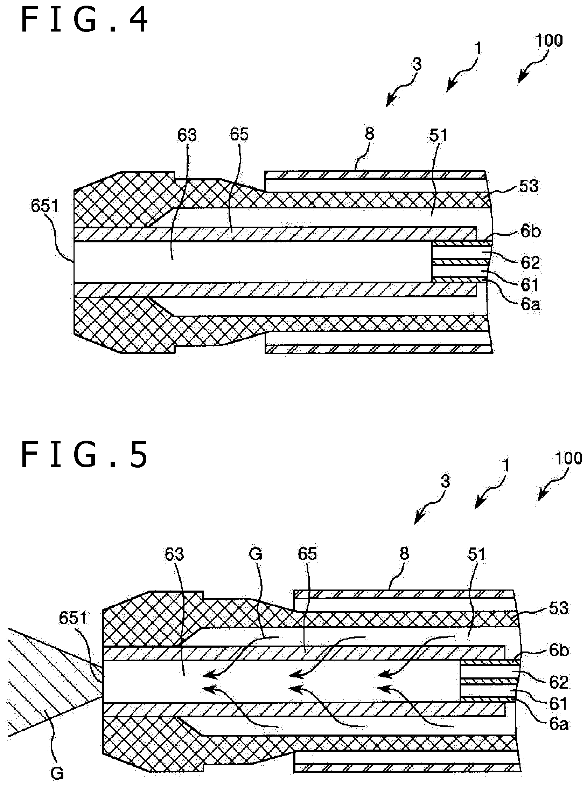

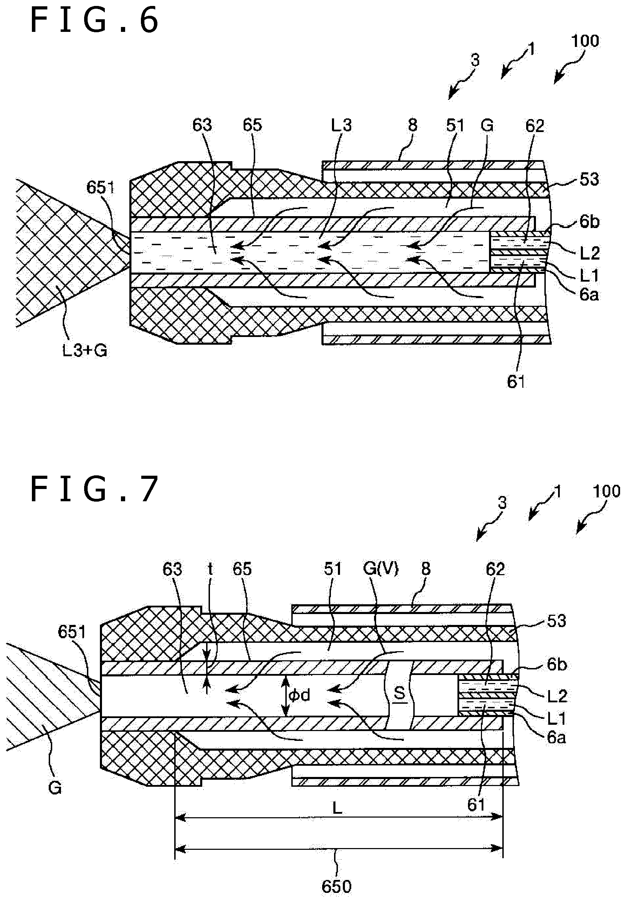

FIG. 1 is a perspective view showing an embodiment of an applicator disclosed herein. FIG. 2 is a longitudinal sectional view of a nozzle possessed by the applicator shown in FIG. 1. FIGS. 3A and 3B are longitudinal sectional views of a check valve possessed by the applicator shown in FIG. 1, wherein FIG. 3A shows a closed state and FIG. 3B shows an open state. FIG. 4 is an enlarged detailed view of a distal portion of the nozzle depicted in FIG. 2, showing variations in an application state with the lapse of time. FIG. 5 is an enlarged detailed view of the distal portion of the nozzle depicted in FIG. 2, showing variations in the application state with the lapse of time. FIG. 6 is an enlarged detailed view of the distal portion of the nozzle depicted in FIG. 2, showing variations in the application state with the lapse of time. FIG. 7 is an enlarged detailed view of the distal portion of the nozzle depicted in FIG. 2, showing variations in the application state with the lapse of time.

Note that for convenience of explanation, the right side in FIGS. 1 to 7 will be referred to as "proximal end (rear)" or "upstream side," and the left side in the figures will be referred to as "distal end (front)" or "downstream side." Besides, in FIGS. 4 to 7, for easier understanding, the dimensions in the lengthwise direction of the nozzle are shown in contracted state, whereas the dimensions in the thickness (diametric size) direction of the nozzle are shown in exaggerated state, so that the ratios between the dimension in the lengthwise direction and the dimension in the thickness (diametric size) direction are different from the actual ratios.

An applicator 100 illustrated in FIG. 1 includes a syringe interlock body 10 and a nozzle 3. As shown in FIG. 6, the applicator 100 is for performing an applying operation of applying a mixed liquid L3 obtained by mixing a first liquid L1 and a second liquid L2 which are different in liquid composition together with a gas G while mixing the first liquid L1 and the second liquid L2. Each of components of the configuration will be described below.

As depicted in FIG. 1, the syringe interlock body 10 is a liquid supply section that collectively supplies the first liquid L1 and the second liquid L2 to the nozzle 3, and includes a syringe 1 a and a syringe 1 b arranged side by side and interlocked to each other. Note that the syringe 1 a functions as a first liquid supply section, and the syringe 1 b functions as a second liquid supply section.

Note that the syringe 1 a is preliminarily filled with the first liquid L1, and the syringe 1 b is preliminarily filled with the second liquid L2.

The first liquid L1 is a solution containing fibrinogen, human blood coagulation factor XIII, aprotinin solution, and additives. Examples of the additives include human serum albumin, glycine, D-mannitol, sodium citrate hydrate, and sodium chloride.

The second liquid L2 is a solution containing thrombin, calcium chloride hydrate, and additives. Examples of the additives include sodium citrate and sodium chloride.

The mixed liquid L3 obtained by mixing the first liquid L1 and the second liquid L2 functions as a living body tissue adhesive.

The syringe 1 a and the syringe 1 b are substantially the same in configuration, and, accordingly, the syringe 1 a will be described below on a representative basis.

The syringe 1 a is composed of a syringe outer tube 2 and a gasket 12.

The syringe outer tube 2 includes a barrel section 21 having a bottomed tubular shape, and a mouth section 22 projectingly formed at a bottom portion constituting a distal wall portion 211 of the barrel section 21.

The barrel section 21 has an inside diameter and an outside diameter which are each constant along the axial direction of the barrel section 21.

In addition, the barrel section 21 of the syringe 1a and the barrel section 21 of the syringe 1 b are interlocked to each other at an intermediate position in regard of the axial direction thereof, through a plate-shaped flange section 23. By this, the positional relation between the syringe 1 a and the syringe 1 b is restricted; in other words, a state in which the syringe 1 a and the syringe 1 b are arranged side by side and interlocked to each other is maintained.

The mouth section 22 is a section which has a tubular shape smaller in thickness (diametric size) than the barrel section 21 and communicates with the barrel section 21. Through the mouth section 22, the first liquid L1 is discharged. Note that the mouth section 22 is disposed in the center of the distal wall portion 211 of the barrel section 21.

In addition, in this embodiment, the outside diameter of the mouth section 22 of the syringe 1 a and the outside diameter of the mouth section 22 of the syringe 1 b are equal.

The material constituting the syringe outer tube 2 is not particularly limited. For example, from the viewpoint of easy molding, the constituent material is preferably a resin material such as polypropylene, cyclic polyolefin, polyesters, and poly(4-methylpentene-1). Note that it is preferable for the constituent material of the syringe outer tube 2 to be substantially transparent, for securing inside visibility.

The gasket 12 is composed of a cylindrical or circular disk-shaped elastic body. The gasket 12 is accommodated in the barrel section 21 and is slidable within the barrel section 21. In addition, a space surrounded by the gasket 12 and the barrel section 21 can be filled with the first liquid L1. With the gasket 12 moved in the distal direction starting from the filled state, the first liquid L1 can be discharged through the mouth section 22.

The material constituting the gasket 12 is not particularly limited. Examples of the constituent material include elastic materials such as various rubber materials, such as silicone rubbers, various thermoplastic elastomers based on polyurethane or the like, and mixtures of them.

Besides, the syringe interlock body 10 further includes a plunger unit 11.

The plunger unit 11 is a member for collectively operating the gaskets 12. The plunger unit 11 includes a plunger section 111 connected to the gasket 12 of the syringe 1 a, a plunger section 112 connected to the gasket 12 of the syringe 1 b, and a flange section 113 as an operating section.

The plunger section 111 is elongate in shape, and its distal portion is connected to the gasket 12 of the syringe 1 a. The plunger section 112 is elongate in shape, and its distal portion is connected to the gasket 12 of the syringe 1 b. The method for this connection is not particularly limited, and examples of the connecting method include screw engagement and fitting.

In addition, the plunger section 112 has a bent portion 114 at an intermediate position in regard of the longitudinal direction thereof, and has a proximal portion bent toward the side of the plunger section 111. Besides, a proximal end of the plunger section 112 is interlocked to a proximal portion of the plunger section 111.

The flange section 113 is plate-like in shape, and the plunger section 111 extends in the distal direction from a distal surface of the flange section 113. At the time of operating the applicator 100, for example, the thumb of one hand can be put on the flange section 113 of the plunger unit 11, and the index finger and the middle finger can be put on the flange section 23 of the syringe outer tube 2.

When a pushing operation of pushing the plunger unit 11 toward the distal side is conducted, the plunger section 111 moves the gasket 12 toward the distal side, whereby the first liquid L1 is discharged through the mouth section 22. In this instance, the plunger section 112 interlocked to the plunger section 111 is also moved toward the distal side together with the plunger section 111, whereby the second liquid L2 is also discharged through the mouth section 22. In other words, the first liquid L1 and the second liquid L2 are simultaneously discharged through the respective mouth sections 22. In addition, since the moving amount of the plunger section 111 toward the distal side is equal to that of the plunger section 112, the plunger section 111 and the plunger section 112 can be prevented from chattering.

As illustrated in FIG. 2, the nozzle 3 includes: a base section 4; a structural body 7 having a double tube structure composed of an outer tube 5 and inner tubes 6a and 6b; and a sheath 8.

The base section 4 is composed of a member having a flat outer shape. The material constituting the base section 4 is not particularly limited; for example, the materials which are the same as or similar to the materials for constituting the syringe outer tube 2 can be used.

The base section 4 is provided with connection sections 41 a and 41 b at a proximal portion thereof. The connection section 41 a is composed of a cylindrically shaped recess, to the inside of which a connection section 911 of a first backflow preventing section 9a to be described later is connected in a liquid-tight manner. Also, the connection section 41 b is composed of a cylindrically shaped recess, to the inside of which a connection section 911 of a second backflow preventing section 9b to be described later is connected in a liquid-tight manner.

In addition, the base section 4 is provided with a connection section 42 at a surface on one side thereof, namely, at a surface which constitutes a lower surface in a condition where the applicator 100 is used. The connection section 42 is composed of a cylindrically shaped recess, to which one end portion of a flexible tube 13 is connected in a liquid-tight manner. The other end portion of this tube 13 is connected with a gas supply unit which is not shown. By this, the gas G can be supplied to the applicator 100.

Besides, at an intermediate portion of the tube 13, a filter 15 accommodated in a housing 14 is arranged. The filter 15 can capture impurities mixed in the gas G, prior to the supply of the gas G to the applicator 100.

The gas G is not particularly limited, and examples thereof include air. In addition, the gas G is preferably a gas in a sterile state, but it may be or may not be in a sterile state.

As shown in FIGS. 1 and 2, the structural body 7 is elongate in shape, and extends in the distal direction from the base section 4. As aforementioned, the structural body 7 is composed of the outer tube 5 and the inner tubes 6a and 6b.

As illustrated in FIG. 2, the inner tube 6a and the inner tube 6b are the same in thickness (diametric size), namely, they are equal in inside diameter and outside diameter.

The inner tube 6a has its proximal portion connected to the mouth section 22 of the syringe 1 a through the backflow preventing section 9. This enables the first liquid L1 to flow through the inner tube 6a. Thus, the inside of the inner tube 6a functions as a first liquid flow path 61 through which the first liquid L1 flows. Also, the inner tube 6b has its proximal portion connected to the mouth section 22 of the syringe 1 b through the backflow preventing section 9. This enables the second liquid L2 to flow through the inner tube 6b. Thus, the inside of the inner tube 6b functions as a second liquid flow path 62 through which the second liquid L2 flows.

Distal portions, or downstream-side portions, of the inner tubes 6a and 6b join each other to form a confluence section 63. As depicted in FIG. 6, the first liquid L1 and the second liquid L2 flow into the confluence section 63 to mix with each other, whereby the mixed liquid L3 is prepared.

The applicator 100 can be divided into: flexible sections 64 where the inner tube 6a and the inner tube 6b respectively define the first liquid flow path 61 and the second liquid flow path 62 that are dependent from each other; and a gas-permeable membrane 65 that defines the confluence section 63 on the distal side of the flexible sections 64.

The flexible sections 64 of the inner tubes 6a and 6b are flexible, and the constituent material thereof is not particularly limited, examples of the applicable constituent material including various thermoplastic elastomers based on polyvinyl chloride, polyurethane or the like.

The gas-permeable membrane 65 is tubular in shape, and its proximal portion is collectively fitted to distal portions of the flexible sections 64 in a liquid-tight manner. In addition, a distal opening portion of the gas-permeable membrane 65 constituting the distal end of the nozzle 3 constitutes a jet port 651 through which the mixed liquid L3 is ejected.

The gas-permeable membrane 65 permeable to the gas G in this way is formed with a multiplicity of minute holes which are not illustrated. Each of the minute holes pierces through the gas-permeable membrane 65 in the thickness direction of the latter. Note that the porosity and the average hole diameter in regard of the gas-permeable membrane 65 are not particularly limited, so long as the flow rate V of the gas G is within a predetermined range which will be described later.

In addition, as depicted in FIG. 7, the average thickness t of the gas-permeable membrane 65 is not particularly limited; for example, the thickness t is preferably 0.2 to 0.8 mm, and more preferably 0.4 to 0.6 mm. This enables the gas G to permeate through the gas-permeable membrane 65 in an assured manner.

Besides, as shown in FIG. 7, the inside diameter φd of the gas-permeable membrane 65 is preferably 0.7 to 1.5 mm, and more preferably 1.0 to 1.2 mm. This enables the uniform mixed liquid L3 to be obtained through assured mixing of the first liquid L1 and the second liquid L2 in the confluence section 63.

The gas-permeable membrane 65 is impermeable to the first liquid L1 and the second liquid L2, that is, the gas-permeable membrane 65 is hydrophobic. This makes it possible to prevent the mixed liquid L3 in the inside from flowing out to the radially outer side, namely, to a gas flow path 51 side through the gas-permeable membrane 65.

Such a gas-permeable membrane 65 is formed from a hydrophobic material, or has a surface treated to be hydrophobic. Examples of the hydrophobic material include polytetrafluoroethylene, tetrafluoroethylene-hexafluoropropylene copolymer, tetrafluoroethylene-perfluoroalkyl vinyl ether copolymer, polychlorotrifluoroethylene, polyvinylidene fluoride, ethylene-tetrafluoroethylene copolymer, ethylene-chlorotrifluoroethylene copolymer, and polypropylene. The gas-permeable membrane 65 is preferably a membrane obtained by a method wherein such a material as just-mentioned is made to be porous by a drawing (stretching or orientation) method, a micro phase separation method, an electron beam etching method, a sintering method, an argon plasma particle method or the like. In addition, the method of making the surface of the gas-permeable membrane 65 hydrophobic is not particularly limited, and examples thereof include a method of coating the surface of the gas-permeable membrane 65 with a hydrophobic material.

Besides, the gas-permeable membrane 65 is tubular in overall shape, which enables the gas G to flow into the confluence section 63 through the gas-permeable membrane 65 from any part in the circumferential direction of the latter. As a result, the gas G can be supplied into the confluence section 63 sufficiently and non-excessively, and, therefore, the mixed liquid L3 ejected is sprayed reliably. Note that as depicted in FIG. 7, when the ejection of the mixed liquid L3 is stopped, the gas G flowing in through the gas-permeable membrane 65 securely blows off the mixed liquid L3 in the confluence section 63 outward. By this, the mixed liquid L3 is prevented from remaining in the confluence section 63. Therefore, the mixed liquid L3 can be prevented from solidifying to clog up the jet port 651. In addition to this, a residual portion of the mixed liquid L3 can be securely prevented from leaking out through the jet port 651.

As illustrated in FIG. 2, the inner tubes 6a and 6b as above extend through the inside of the outer tube 5. The outer tube 5 has its proximal portion supported by the base section 4, and communicates with the tube 13 connected to the base section 4. By this, the gas G can be supplied into the outer tube 5. In addition, a gap is formed between the outer tube 5 and the inner tubes 6a and 6b, and the gas G can flow through the gap. Thus, the outer tube 5 functions as the gas flow path 51 through which the gas G flows. Besides, as aforementioned, the gas G flows into the confluence section 63 through the gas-permeable membrane 65.

In addition, in the applicator 100, the outer tube 5 can be divided into a hard section 52 and a flexible section 53 located on the distal side of the hard section 52.

The hard section 52 is a section which accounts for not less than 60% of the outer tube 5 and is formed from one of various metallic materials such as stainless steel, aluminum, copper, and copper alloys. By this, the posture of the nozzle 3 as a whole can be maintained, that is, the nozzle 3 as a whole can be prevented from bending. Note that the sheath 8 also is preferably formed from a material the same as or similar to that of the hard section 52.

The flexible section 53 is formed, for example, from one of various thermoplastic elastomers based on polyvinyl chloride, polyurethane or the like, like the flexible section 64 of the inner tube 6a. The flexible section 53 is provided with a bending tendency such as to bend in a natural state where no external force is exerted thereon. When the tubular sheath 8 covering the outer tube 5 is moved forward in the direction of arrow A in FIG. 2, the flexible section 53 can be straightened into a rectilinear shape, so that the jet port 651 is oriented forward. In addition, when the sheath 8 is moved rearward in the direction of arrow B in FIG. 2, the flexible section 53 is released from the straightening and curved, so that the jet port 651 is oriented obliquely forward or sideways, as indicated by alternate long and two short dashes line in FIG. 2. Thus, in the applicator 100, the orientation of the jet port 651 can be changed according to the position of a target part, by a moving operation of the sheath 8.

As shown in FIGS. 1 to 3, the backflow preventing section 9 includes the first backflow preventing section 9a for preventing backflow of the first liquid L1, and the second backflow preventing section 9b for preventing backflow of the second liquid L2. The first backflow preventing section 9a is provided between the syringe 1 a and the base section 4, and the second backflow section 9b is provided between the syringe 1 b and the base section 4.

The first backflow preventing section 9a and the second backflow preventing section 9b are the same in configuration, and, accordingly, the first backflow preventing section 9a will be described on a representative basis.

The first backflow preventing section 9a includes a housing 91, and a check valve 92 provided inside the housing 91.

The housing 91 is composed of a cylindrical hollow body, which is provided with the connection section 911 formed to project toward the distal side, and a connection section 912 formed to project toward the proximal side.

The connection section 911 and the connection section 912 are each hollow cylindrical in shape. The connection section 911 is inserted in the connection section 41 a, whereas the mouth section 22 of the syringe 1 a is inserted in the connection section 912. By this configuration, the syringe 1 a and the base section 4 can be connected to each other through the housing 91. Therefore, the first liquid L1 discharged from the mouth section 22 can flow into the first liquid flow path 61 through the inside of the housing 91.

Note that in an applicator 1, the inside of the housing 91 is also included in the first liquid flow path 61. Besides, also in the second backflow preventing section 9b, similarly, the inside of the housing 91 is included in the second liquid flow path 62.

The check valve 92 has a diaphragm 921. The diaphragm 921 is composed of a membrane member capable of opening and closing the first liquid flow path 61. The diaphragm 921 is configured to be movable between a first position P1 depicted in FIG. 3A and a second position P2 shown in FIG. 3B.

When positioned in the first position P1 as shown in FIG. 3A, the diaphragm 921 closes the connection section 912. By this, in the first liquid flow path 61 is established a closed state in which the first liquid L1 is inhibited from flowing.

On the other hand, when positioned in the second position P2 as depicted in FIG. 3B, the diaphragm 921 is in the state of being separate from the connection section 912. By this, a gap 200 is formed between the connection section 912 and the diaphragm 921, so that the first liquid L1 can flow into the first liquid flow path 61 through the gap 200.

Note that the diaphragm 921 may be biased toward the proximal side, or the upstream side, by a biasing section which is not illustrated. The biasing section is not particularly limited; for example, a leaf spring or a coil spring can be used.

Where such a first backflow preventing section 9a is used, when a pushing operation of the plunger section 111 is performed starting from the closed state of the diaphragm 921 shown in FIG. 3A, the first liquid L1 pushes the diaphragm 921 toward the distal side, whereby the open state is established in the applicator 1 as shown in FIG. 3B. As a result, the first liquid L1 can flow through the first liquid flow path 61 by way of the gap 200.

When the pushing operation of the plunger section 111 is stopped from the state shown in FIG. 3B, a backflow force of the first liquid L1 tending to flow back or a biasing force of the biasing section causes the diaphragm 921 to move toward the upstream side, to close the connection section 912. This results in the closed state, whereby backflow of the first liquid L1 can be prevented.

Here, in the applicator 1, the mixed liquid L3 flows through the confluence section 63, as aforementioned. When the gas G flows into the confluence section 63 from a lateral side, a pressure is exerted on the mixed liquid L3 in the confluence section 63. In this instance, a pressure may be exerted on the mixed liquid L3 in a direction for backflow toward the upstream side. If this backflow of the mixed liquid L3 causes the mixed liquid L3 to flow into the first liquid flow path 61 and the second liquid flow path 62, the first liquid flow path 61 and the second liquid flow path 62 may be clogged.

In the applicator 1, backflow of the first liquid L1 in the first liquid flow path 61 is prevented by the first backflow preventing section 9a, and backflow of the second liquid L2 in the second liquid flow path 62 is prevented by the second backflow preventing section 9b, as aforementioned. By this, the mixed liquid L3 can be prevented from flowing back to enter into the first liquid flow path 61 and the second liquid flow path 62. Specifically, even if the mixed liquid L3 tends to enter into the first liquid flow path 61 and the second liquid flow path 62, the first liquid L1 already present in the first liquid flow path 61 and the second liquid L2 already present in the second liquid flow path 62 inhibit the mixed liquid L3 from entering into the first and second liquid flow paths 61 and 62.

With the configuration as above, in the applicator 1, the first liquid flow path 61 and the second liquid flow path 62 can be prevented from being clogged. As a result, after an applying operation is stopped, the next applying operation can be performed smoothly and reliably.

Furthermore, in the applicator 1, it is possible to not only prevent clogging in the first liquid flow path 61 and the second liquid flow path 62 but also prevent clogging at the jet port 651. This will be described below.

When the applying operation of the applicator 1 is stopped, the mixed liquid L3 is remaining in the confluence section 63. The residual portion of the mixed liquid L3 would cause clogging in the confluence section 63, particularly, at the jet port 651. In order to prevent the clogging at the jet port 651, the mixed liquid L3 remaining in the confluence section 63 should be discharged through the jet port 651. In the applicator 1, the mixed liquid L3 thus remaining can be reliably discharged through the jet port 651 by the gas G; in this case, in order to ensure reliable discharge, the pressure of the gas G jetted out together with the mixed liquid L3 should be optimized.

In view of this, as illustrated in FIG. 7, the length L of the gas-permeable membrane 65 as the overall length in the direction in which the mixed liquid L3 flows through the confluence section 63 is set to be 5.0 to 39.0 mm, and the flow rate V of the gas G permeating through the gas-permeable membrane 65 is set to be 0.3 to 2.4 L/minute in the applicator 1. By this, the mixed liquid L3 remaining in the confluence section 63 can be discharged through the jet port 651. As a result, after the applying operation is stopped, the next applying operation can be carried out smoothly and assuredly. Furthermore, with the length L and the flow rate V set to within the numerical value ranges, the layer of the mixed liquid L3 applied shows excellent pressure resistance at the target part, specifically, in the living body. As a result, the mixed liquid L3 can stay at the target part for a sufficiently long period of time, thereby functioning as an excellent living body tissue adhesive.

Note that, as depicted in FIG. 7, the "length L" herein refers to the overall length of that part of the gas-permeable membrane 65 through which the gas G can flow into the confluence section 63, namely, an effective permeation region 650 as that part of the gas-permeable membrane 65 which fronts on the gas flow path 51.

In the applicator 1, the aforementioned effect cannot be sufficiently obtained if at least one of the length L and the flow rate V falls outside the numerical value range.

For example, when the length L is too short or the flow rate V is too low, the pressure of the gas G ejected through the jet port 651 tends to be excessively low. In this case, the mixed liquid L3 in the confluence section 63 may be blown off insufficiently, so that the mixed liquid L3 may remain in the confluence section 63. Furthermore, if the pressure of the gas G ejected through the jet port 651 becomes too low, the mixed liquid L3 ejected through the jet port 651 may drip down from the jet port 651. In this case, the thickness of the layer of the mixed liquid L3 applied to the target part may become insufficient. As a result, the layer of the mixed liquid L3 applied may show an insufficient pressure resistance in the living body, and may become poor in the function as a living body tissue adhesive.

Besides, for example, when the length L is too long or the flow rate V is too high, the pressure of the gas G ejected through the jet port 651 tends to be excessively high. In this case, since the mixed liquid L3 is applied over a wide range, the mixed liquid L3 is applied also to the vicinity of the target part, so that the thickness of the layer of the mixed liquid L3 applied to the target part may be reduced. As a result, the layer of the mixed liquid L3 applied may exhibit an insufficient pressure resistance in the living body, and may become poor in the function as a living body tissue adhesive.

As described above, in the applicator 1, the aforementioned effect can be obtained when the length L of the gas-permeable membrane 65 is set to be 5.0 to 39.0 mm and the flow rate V of the gas G permeating through the gas-permeable membrane 65 is set to be 0.3 to 2.4 L/minute. In this case, the length L of the gas-permeable membrane 65 is preferably 10.0 to 35.0 mm, and the flow rate V of the gas G permeating through the gas-permeable membrane 65 is preferably 0.3 to 2.4 L/minute.

In addition, the flow rate V of the mixed liquid L3 is preferably 1.2 to 1.8 L/minute. By this setting, the mixed liquid L3 remaining in the confluence section 63 can be securely discharged through the jet port 651, and it is ensured that the layer of the mixed liquid L3 applied shows excellent pressure resistance in the living body.

Besides, as shown in FIG. 7, the surface area of the gas-permeable membrane 65, specifically, the area S of an outer peripheral surface of the gas-permeable membrane 65 is preferably 10 to 250 mm², more preferably 15 to 160 mm². Under this condition, the gas G can permeate through the gas-permeable membrane 65 in a reliable manner. Note that the area S of the outer peripheral surface herein refers to the area of the outer peripheral surface of the effective permeation region 650.

The first liquid L1 preferably contains 75 to 85 mg of fibrinogen and 65 to 85 units of blood coagulation factor XIII per 1 mL, and the second liquid L2 preferably contains 220 to 280 units of thrombin per 1 mL. The mixed liquid L3 with the first liquid L1 and the second liquid L2 prepared in such states is excellent as a living body tissue adhesive.

Besides, where the volume of the first liquid L1 inside the gas-permeable membrane 65 is V1 and the volume of the second liquid L2 inside the gas-permeable membrane 65 is V2, the mixing ratio V1/V2 preferably falls within the range from 0.8 to 1.2, more preferably the range from 0.9 to 1.1. Under this condition, the mixed liquid L3 is excellent as a living body tissue adhesive.

Operations of the applicator 100 will be described below referring to FIGS. 4 to 7.
[1] First, as depicted in FIG. 4, the applicator 100 is prepared. Then, prior to an applying operation, a valve of a gas cylinder is opened, to preliminarily supply the gas G to the applicator 100. By this, as shown in FIG. 5, the gas G in the nozzle 3 sequentially passes through the gas flow path 51 and the confluence section 63, to be ejected through the jet port 651.
   Note that the first liquid flow path 61 is not yet filled with the first liquid L1, and the second liquid flow path 62 is not yet filled with the second liquid L2.
[2] Next, the index finger and the middle finger of one hand are put on the flange section 23 of the syringe outer tubes 2, and the thumb is put on the flange section 113 of the plunger unit 11. Thereafter, the jet port 651 of the nozzle 3 is oriented toward a target part. Then, in this condition, a force is applied with the thumb to push the plunger unit 11 toward the distal side, thereby performing an applying operation. By this, as illustrated in FIG. 6, the first liquid flow path 61 is supplied with the first liquid L1, and the second liquid flow path 62 is supplied with the second liquid L2.
   In addition, in the confluence section 63, the first liquid L1 and the second liquid L2 having flowed thereto mix with each other, to be the mixed liquid L3. The mixed liquid L3 is ejected through the jet port 651 together with the gas G in a spray form (atomized form), to be applied to the target part.
[3] After a predetermined amount of the mixed liquid L3 is applied to the target part, the pushing force on the plunger unit 11 is relaxed, to temporarily stop the applying operation. In this instance, the gas G is continuously flowing into the confluence section 63.

By this gas G, the first liquid L1 in the first liquid flow path 61 is pushed back toward the upstream side. However, the backflow preventing section 9 prevents further backflow of the first liquid L1 within the first liquid flow path 61, as aforementioned. Note that backflow of the second liquid L2 is also prevented in a similar manner. By this, the first liquid flow path 61 and the second liquid flow path 62 can be prevented from being clogged.

Furthermore, in the applicator 1, the length L of the gas-permeable membrane 65 is 5.0 to 39.0 mm and the flow rate V of the gas G permeating through the gas-permeable membrane 65 is 0.3 to 2.4 L/minute, as aforementioned. By this, the mixed liquid L3 remaining in the confluence section 63 can be discharged through the jet port 651, so that clogging can be prevented from occurring.

Thus, due to the prevention of clogging in the first liquid flow path 61 and the second liquid flow path 62 and the prevention of clogging at the jet port 651, the next applying operation can be performed more reliably.

Furthermore, with the length L and the flow rate V set to within the aforementioned numerical value ranges, the layer of the mixed liquid L3 shows excellent pressure resistance at the target part. As a result, the mixed liquid L3 can stay at the target part for a sufficiently long period of time, and therefore functions as an excellent living body tissue adhesive.

While the applicator disclosed herein has been described above with reference to the embodiment shown in the drawings, the present disclosure is not limited to the embodiment. The components of the applicator may be replaced by arbitrary configurations capable of exhibiting the same or equivalent functions to the original. Besides, arbitrary components may be added to the above-described ones.

Besides, while a case where the first liquid L1 is a solution containing fibrinogen and the second liquid L2 is a solution containing thrombin has been described in the embodiment, this is not restrictive. Thus, the first liquid L1 may be a solution containing thrombin, and the second liquid L2 may be a solution containing fibrinogen.

In addition, while a case where the mixed liquid functions as a living body tissue adhesive has been described in the embodiment, this is not restrictive. For example, the mixed liquid may function as an anti-adhesion material.

### Examples

Specific examples of the present disclosure will be described below. The present disclosure is not to be limited to the following Examples.

### 1. Fabrication of Applicator

### (Example 1)

An applicator as shown in FIGS. 1 to 7 was prepared.

In addition, as a gas-permeable membrane, a "poly tetrafluoroethylene (PTFE) Porous Tube" made by Chukoh Chemical Industries, Ltd. was used, with a length L of 10.0 mm. The area S of the outer peripheral surface of the gas-permeable membrane was 35 mm². The gas-permeable membrane had an average thickness t of 0.5 mm and an inside diameter φd of 1.1 mm.

Besides, Vial 1 and Vial 2 of BOLHEAL made by The Chemo-Sero-Therapeutic Research Institute were mixed with each other, to form a first liquid, which was placed in the syringe 1 a. The Vial 1 contains human fibrinogen and human blood coagulation factor XIII, and further contains human serum albumin, glycine, D-mannitol, sodium citrate hydrate, and sodium chloride as additives. The Vial 2 contains Japanese Pharmaceutical Codex aprotinin solution, and further contains sodium chloride as an additive.

In addition, Vial 3 and Vial 4 of BOLHEAL made by The Chemo-Sero-Therapeutic Research Institute were mixed with each other, to form a second liquid, which was placed in the syringe 1 b. The Vial 3 contains Japanese Pharmacopoeia thrombin, and further contains sodium citrate hydrate and sodium chloride as additives. The Vial 4 contains Japanese Pharmacopoeia calcium chloride hydrate.

The amount of human fibrinogen in 1 mL of the first liquid was 80 mg/mL, and the amount of human blood coagulation factor XIII was 75 units (where the activity of blood coagulation factor XIII contained in 1 mL of human plasma was taken as 1 unit). Besides, the concentration of Japanese Pharmaceutical Codex aprotinin solution in 1 mL of the first liquid was 1,000 KIE/mL (where the amount for halving the efficacy of 2 units of kallidinogenase in two hours at pH 8 and room temperature was taken as 1 KIE).

In addition, the amount of Japanese Pharmacopoeia thrombin in 1 mL of the second liquid was 250 units. The concentration of Japanese Pharmacopoeia calcium chloride hydrate was 5.9 mg/mL.

Besides, the mixing ratio V1/V2 of the first liquid and the second liquid in the mixed liquid was set to 1.0.

In this Example, an air-containing gas was used as the gas, and an applying operation was conducted in such a manner that the flow rate of the gas G permeating through the gas-permeable membrane 65 during the applying operation was 0.5 L/minute.

### (Example 2)

An applicator of Example 2 was obtained in the same manner as in Example 1, except that the length L of the gas-permeable membrane was 20.0 mm and the area S of the outer peripheral surface was 69 mm². In Example 2, the applying operation was conducted such that the flow rate V of the gas was 0.6 L/minute.

### (Example 3)

An applicator of Example 3 was obtained in the same manner as in Example 1, except that the length L of the gas-permeable membrane was 20.0 mm and the area S of the outer peripheral surface was 69 mm². In Example 3, the applying operation was conducted such that the flow rate V of the gas was 1.0 L/minute.

### (Example 4)

An applicator of Example 4 was obtained in the same manner as in Example 1, except that the length L of the gas-permeable membrane was 20.0 mm and the area S of the outer peripheral surface was 69 mm². In Example 4, the applying operation was conducted such that the flow rate V of the gas was 1.2 L/minute.

### (Example 5)

An applicator of Example 5 was obtained in the same manner as in Example 1, except that the length L of the gas-permeable membrane was 15.0 mm and the area S of the outer peripheral surface was 52 mm². In Example 5, the applying operation was conducted such that the flow rate V of the gas was 1.2 L/minute.

### (Example 6)

An applicator of Example 6 was obtained in the same manner as in Example 1, except that the length L of the gas-permeable membrane was 35.0 mm and the area S of the outer peripheral surface was 121 mm². In Example 6, the applying operation was conducted such that the flow rate V of the gas was 1.2 L/minute.

### (Example 7)

An applicator of Example 7 was obtained in the same manner as in Example 1, except that the length L of the gas-permeable membrane was 20.0 mm and the area S of the outer peripheral surface was 69 mm². In Example 7, the applying operation was conducted such that the flow rate V of the gas was 1.7 L/minute.

### (Example 8)

An applicator of Example 8 was obtained in the same manner as in Example 1, except that the length L of the gas-permeable membrane was 20.0 mm and the area S of the outer peripheral surface was 69 mm². In Example 8, the applying operation was conducted such that the flow rate V of the gas was 2.0 L/minute.

### (Comparative Example 1)

An applicator of Comparative Example 1 was obtained in the same manner as in Example 1, except that the length L of the gas-permeable membrane was 10.0 mm. In Comparative Example 1, the applying operation was conducted such that the flow rate V of the gas was 0.2 L/minute.

### (Comparative Example 2)

An applicator of Comparative Example 2 was obtained in the same manner as in Example 1, except that the length L of the gas-permeable membrane was 20.0 mm and the area S of the outer peripheral surface was 69 mm². In Comparative Example 2, the applying operation was conducted such that the flow rate V of the gas was 0.3 L/minute.

### (Comparative Example 3)

An applicator of Comparative Example 3 was obtained in the same manner as in Example 1, except that the length L of the gas-permeable membrane was 40.0 mm and the area S of the outer peripheral surface was 138 mm². In Comparative Example 3, the applying operation was conducted such that the flow rate V of the gas was 2.5 L/minute.

### <Clogging Test>

The mixed liquid is jetted together with a gas by the applicator for 10 seconds.

This jetting is the first jetting. Then, the supply of the mixed liquid is stopped, that is, the first jetting is stopped, and, after 60 minutes, the application is restarted. The jetting in this instance is the second jetting. While using the same cycle, the jetting and the restarting were repeated, and how many times the jetting could be conducted was measured.

### <Pressure Resistance Test>

First, a skin of a rabbit was extracted in a circular shape with a diameter of 20 mm, and a multiplicity of circular through-holes with a diameter of 0.1 mm were formed in the rabbit skin thus extracted. Note that the through-holes were formed in a density of 0.03 holes/mm².

Next, using the applicator, the mixed liquid was applied to one side of the rabbit skin.

Then, air was blown from the other side of the rabbit skin, the pressure of air thus blown was gradually raised, and the pressure (mmHg) at which the layer of the mixed liquid on the one side was broken was measured.

The evaluation results for the applicators obtained in Examples and Comparative Examples are set forth in Table 1 below.

**Table 1**

| | | Examples | | | | | | | | Comparative Examples | | |
|---|---|---|---|---|---|---|---|---|---|---|---|---|
| | | 1 | 2 | 3 | 4 | 5 | 6 | 7 | 8 | 1 | 2 | 3 |
| First liquid | Human fibrinogen (mg/mL) | 80 | 80 | 80 | 80 | 80 | 80 | 80 | 80 | 80 | 80 | 80 |
| | Human blood coagulation factor XIII (units/mL) | 75 | 75 | 75 | 75 | 75 | 75 | 75 | 75 | 75 | 75 | 75 |
| Second liquid | Japanese Pharmacopoeia thrombin (units/mL) | 250 | 250 | 250 | 250 | 250 | 250 | 250 | 250 | 250 | 250 | 250 |
| Mixed liquid | Mixing ratio V1/V2 | 1.0 | 1.0 | 1.0 | 1.0 | 1.0 | 1.0 | 1.0 | 1.0 | 1.0 | 1.0 | 1.0 |
| Gas | Flow rate V (L/minute) | 0.5 | 0.6 | 1.0 | 1.2 | 1.2 | 1.2 | 1.7 | 2.0 | 0.2 | 0.3 | 2.5 |
| Gas-permeable membrane | Length L (mm) | 10.0 | 20.0 | 20.0 | 20.0 | 15.0 | 35.0 | 20.0 | 20.0 | 10.0 | 20.0 | 40.0 |
| | Area of outer peripheral surface (mm²) | 35 | 69 | 69 | 69 | 52 | 121 | 69 | 69 | 35 | 69 | 138 |
| | Average thickness (mm) | 0.5 | 0.5 | 0.5 | 0.5 | 0.5 | 0.5 | 0.5 | 0.5 | 0.5 | 0.5 | 0.5 |
| | Inside diameter (mm) | 1.1 | 1.1 | 1.1 | 1.1 | 1.1 | 1.1 | 1.1 | 1.1 | 1.1 | 1.1 | 1.1 |
| Evaluation | Clogging test (times) | 3 | 2 | 3 | 3 | - | - | 4 | 2 | 1 | 2 | 1 |
| | Pressure resistance (mmHg) | 347 | 236 | - | 385 | 235 | 323 | 346 | - | - | 189 | - |

When an applicator is put to practical use, an applying operation may be stopped and may thereafter be restarted. In view of this, the applicators with which the applying operation could be carried out at least twice were evaluated to be acceptable (to pass the test). As shown in Table 1, the applicators obtained in Examples 2 and 8 could be used twice for the applying operation. In addition, the applicators obtained in Examples 1,3,4, and 7 could be used three or more times for the applying operation. Thus, it was verified that the applicators obtained in Examples are less liable to be clogged, as compared to the applicators obtained in Comparative Examples.

In addition, the mixed liquid applied by the applicator receives a pressure of approximately 120 mmHg in a living body; therefore, the layer of the mixed liquid applied should have a pressure resistance in excess of this pressure value, specifically, a pressure resistance of not less than approximately 200 mmHg. As shown in Table 1, the layers of the mixed liquid applied by the applicators of Examples 2 and 5 showed a pressure resistance higher than 200 mmHg. Further, the layers of the mixed liquid applied by the applicators of Examples 1,4, 6 and 7 showed a pressure resistance further higher than 200 mmHg. Thus, it was verified that the layers of the mixed liquid applied by the applicators of Examples were superior in pressure resistance than the layers of the mixed liquid applied by the applicators of Comparative Examples.

Having described the preferred embodiment of the present disclosure, it is to be understood that the disclosure is not limited to the embodiment and that various changes and modifications could be effected therein by one skilled in the art without departing from the spirit or scope of the disclosure as defined in the appended claims.

## Claims

1. An applicator for ejecting together with a gas a mixed liquid obtained by mixing a first liquid and a second liquid which are different from each other in composition, the applicator comprising:
a first liquid flow path through which the first liquid flows;
a second liquid flow path through which the second liquid flows;
a gas flow path through which the gas flows;
a confluence section in which the first liquid and the second liquid merge with each other to form the mixed liquid, at least part of a wall portion defining the confluence section being composed of a gas-permeable membrane which is impermeable to the mixed liquid and permeable to the gas; and
a backflow preventing section that prevents backflow of the first liquid and prevents backflow of the second liquid.

2. The applicator according to claim 1, wherein the backflow preventing section includes check valves which are each provided in the first liquid flow path and the second liquid flow path.

3. The applicator according to claim 2, further comprising:
a first liquid supply section that communicates with the first liquid flow path and supplies the first liquid into the first liquid flow path; and
a second liquid supply section that communicates with the second liquid flow path and supplies the second liquid into the second liquid flow path,
wherein the check valves are each provided in a boundary section between the first liquid flow path and the first liquid supply section and in a boundary section between the second liquid flow path and the second liquid supply section.

4. The applicator according to claim 2, wherein the check valve has a diaphragm that is openable and closable.

5. The applicator according to claim 4, wherein the check valve has a biasing section that biases the diaphragm.

6. The applicator according to claim 1,
wherein the length of the gas-permeable membrane in a direction in which the mixed liquid flows through the confluence section is 5.0 to 39.0 mm, and
the flow rate of the gas permeating through the gas-permeable membrane is 0.3 to 2.4 L/minute.

7. The applicator according to claim 1, wherein the thickness of the gas-permeable membrane is 0.2 to 0.8 mm.

8. The applicator according to claim 1, wherein the gas-permeable membrane is tubular in shape with an inside diameter of 0.7 to 1.5 mm.

9. The applicator according to claim 1, wherein an outer surface of the gas-permeable membrane has a surface area of 10 to 250 mm².
